# FASCICULE DE BREVET EUROPEEN

(11) **EP 1 099 716 B1**
(45) Date de publication et mention de la délivrance du brevet: **01.03.2006**
(21) Numéro de dépôt: 00402928.6
(22) Date de dépôt: 23.10.2000
(51) Int. Cl.: A61K 8/34, A61K 8/41, A61K 8/49, A61K 8/60, A61K 8/63, A61K 8/67, A61K 8/81, A61Q 1/02, A61Q 1/06, A61Q 1/10, A61Q 3/02, A61Q 5/00, A61Q 19/00, A61Q 19/02, C08F 246/00

(54) **Composition, notamment cosmétique, comprenant un copolymère sur lequel est fixé au moins un azurant optique**
Zusammensetzung, insbesondere für Kosmetik, mit einem Copolymer das modifiziert ist mit einem optischen Aufheller
Composition, particularly for cosmetics, containing a copolymer modified with an optical brightener

(30) Priorité: 10.11.1999 FR 9914150
(43) Date de publication de la demande: 16.05.2001
(73) Titulaire: L'OREAL, 75008 Paris (FR)
(72) Inventeur: Dauga, Christophe, 92300 Levallois-Perret (FR); Chevalier, Véronique, 94400 Villecresnes (FR)
(74) Mandataire: Renard, Emmanuelle

(56) Documents cités:
- EP-A- 0 894 798
- EP-A- 0 962 224
- FR-A- 2 741 261

## Description

L'invention a pour objet une composition pour application topique comprenant, dans un milieu physiologiquement acceptable, au moins un copolymère comportant au moins un azurant optique fixé sur la chaîne du copolymère par une liaison covalente. L'invention a également pour objet les utilisations de cette composition dans le soin et le maquillage de la peau, des phanères et/ou des muqueuses.

Il est fréquent que les personnes ayant une peau colorée, voire foncée, désirent s'éclaircir la peau et utilisent dans ce but des compositions cosmétiques ou dermatologiques contenant des agents de blanchiment.

Les substances les plus utilisées comme agent de blanchiment sont l'hydroquinone et ses dérivés, l'acide kojique et ses dérivés, l'acide azélaïque, l'arbutine et ses dérivés, seuls ou en association avec d'autres actifs.

De telles compositions comprenant des agents de blanchiment sont également utilisées par les personnes dont la peau présente des dyschromies, ces dyschromies pouvant être d'origines diverses : âge (tâches de vieillesse), exposition aux UV, masque de grossesse, pathologie de la peau, etc.

Toutefois, ces agents ne sont pas dépourvus d'inconvénients. En particulier, il est nécessaire de les utiliser de façon prolongée et en des quantités élevées pour observer un effet de blanchiment de la peau. On n'observe pas un effet immédiat à l'application de compositions les comprenant.

En outre, l'hydroquinone et ses dérivés sont utilisés en quantité efficace pour voir apparaître un effet blanchissant. Or, l'hydroquinone est connue pour sa cytotoxicité vis-à-vis du mélanocyte.

Par ailleurs, l'acide kojique et ses dérivés présentent l'inconvénient d'être coûteux et de ne pouvoir, pour cette raison, être utilisés en quantité importante dans des produits à large diffusion commerciale.

Il est connu d'utiliser des compositions cosmétiques susceptibles d'uniformiser le teint, et éventuellement de conférer un aspect blanc immédiat, ces compositions étant constituées de poudres dispersées dans un liant. Les poudres sont généralement des pigments blancs ou colorés suivant l'effet souhaité et/ou des charges de formes différentes (lamellaires, sphériques) suivant l'effet recherché. L'uniformisation du teint est obtenue essentiellement grâce au pouvoir couvrant apporté par les pigments et les charges.

L'inconvénient de telles compositions est que l'estompage des défauts de la peau est apporté par le pouvoir couvrant des compositions. La peau ainsi maquillée perd son aspect naturel du fait du manque de transparence de ces compositions.

Pour remédier à ces inconvénients, il a été proposé dans la demande EP-A-0 962 224 au nom de la Demanderesse de remplacer les charges ci-dessus par des azurants optiques tels que les dérivés du stilbène, les dérivés coumariniques, les dérivés oxazole et benzoxazole et les dérivés imidazole. De tels composés sont par exemple disponibles sous les dénominations commerciales Tinopal SOP® et Uvitex OB® auprès de la société CIBA GEIGY. Les azurants optiques permettent, lorsqu'ils sont incorporés dans des compositions cosmétiques et/ou dermatologiques, d'obtenir un teint blanc, uniforme, homogène, d'aspect naturel, ces compositions présentant une transparence satisfaisante après application sur la peau.

On rappelle que les azurants optiques sont des agents de blanchiment par voie optique, constitués par des composés chimiques dotés de propriétés de fluorescence, qui absorbent dans l'ultraviolet (absorption maximale à une longueur d'onde inférieure à 400 nm) et réémettent de l'énergie par fluorescence pour une longueur d'onde comprise entre 380 nm et 830 nm. Une émission d'énergie comprise entre 400 nm et 480 nm résulte en une émission dans le bleu du domaine visible, ce qui contribue, lorsque cette émission a lieu sur la peau, à la blanchir visuellement.

On connaît en outre de la demande de brevet française publiée sous le numéro FR-2 741 261 des compositions cosmétiques comprenant des agents fluorescents d'avivage également connus comme azurants optiques. Ces agents présentent l'avantage d'intensifier l'éclat et d'aviver les teintes des compositions cosmétiques les comprenant à l'application sur la peau ou les cheveux. Il est en particulier décrit des compositions capillaires et de maquillage (mascara, vernis-à-ongles, rouge-à-lèvres, poudres).

La Demanderesse a maintenant découvert qu'un azurant optique particulier, ayant des propriétés de fluorescence supérieures à celles des azurants connus, pouvait avantageusement être utilisé pour obtenir un blanchiment immédiat et/ou améliorer l'éclat de la peau de la peau, des muqueuses et des phanères.

Cet azurant optique particulier, ainsi que son mode de préparation, sont décrits dans la demande FR 99/10942.

Il s'agit d'un copolymère comportant au moins un azurant optique fixé sur la chaîne du copolymère par une liaison covalente.

On a déjà envisagé de réaliser des polymères et copolymères comportant des azurants optiques afin de blanchir les polymères et d'obtenir des produits blancs résistant aux lavages et aux solvants, pour diverses applications, notamment pour le blanchiment des textiles, du papier et des matières plastiques, par exemple pour la fabrication d'objets d'usage courant tels que des jouets, des articles de papeterie, etc.

Il est également connu de fixer des azurants optiques sur des polymères ou copolymères pour éviter leur dispersion dans divers milieux. On pourra ainsi se référer à I. Grabchev et T. Philipova, Die Angewandte Makromolekulare Chemie, 263, 1998, 1-4, page 4508 et à T. N. Konstantinova et I. K. Grabchev, Polymer International, 43, 1997, pp. 39-44.

Il n'a toutefois jamais été proposé d'utiliser des polymères et copolymères comportant des azurants optiques pour une application topique sur la peau, les muqueuses ou les phanères.

La présente invention a ainsi pour objet une composition pour application topique comprenant, dans un milieu physiologiquement acceptable, au moins un copolymère comportant au moins un azurant optique fixé sur la chaîne du copolymère par une liaison covalente.

Ce copolymère particulier confère avantageusement à la composition selon l'invention une émission de fluorescence d'intensité lumineuse optimale dans la région violet-bleu du spectre visible et peut être fabriqué par des procédés faciles à mettre en oeuvre à partir de monomères polymérisables solubles dans des solvants organiques.

De plus, le fait que l'azurant optique soit fixé par une liaison covalente sur la chaîne polymère empêche sa libération dans le milieu environnant, ce qui le rend compatible avec divers milieux hydrophiles ou hydrophobes. Cette fixation le rend ainsi insensible aux réactions chimiques et le rend compatible avec d'autres colorants, si nécessaire.

Selon une forme d'exécution préférée de l'invention, le copolymère est formé de motifs A et de motifs B comportant un azurant optique C lié de façon covalente aux motifs B, et il comprend de 4 à 30% en poids d'azurant optique C par rapport au poids du copolymère total.

Il a en effet été découvert que l'intensité lumineuse de l'émission fluorescente est maximale quand on limite à une plage précise la teneur en azurant optique du copolymère. Elle est en particulier supérieure à celle observée pour les copolymères décrits dans les deux publications mentionnées précédemment. En revanche, une teneur en azurant optique du copolymère supérieure à 30% en poids peut conduire à une extinction ou "quenching" du phénomène de fluorescence.

L'azurant optique fixé dans ce copolymère peut être choisi parmi les azurants optiques connus et utilisés jusqu'à présent comme blanchissants.

On peut utiliser en particulier un azurant optique choisi dans le groupe constitué :
1) des bis(benzoxazol-2-yl) de formule (I) : 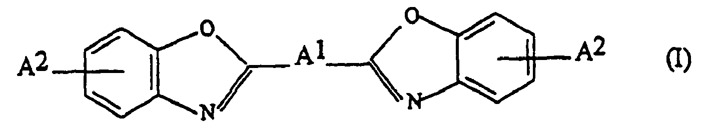
   dans laquelle A¹ est un groupe aromatique, hétérocyclique ou alcoylène, et A² est un atome d'hydrogène ou un groupe alkyle;
2) des coumarines de formule (II): 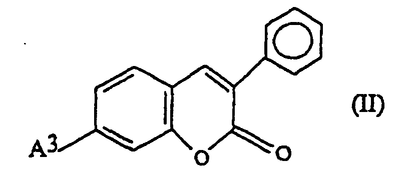
   dans laquelle A³ est est un groupe hétérocyclique ;
3) des bis(styryl)biphényle de formule (III) : 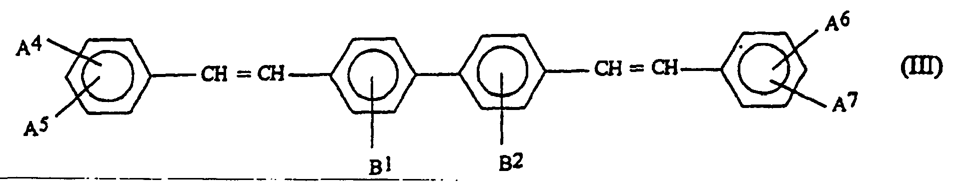
   dans laquelle A⁴, A⁵, A⁶, A⁷, B¹ et B², qui peuvent être identiques ou différents, représentent indépendamment un atome d'hydrogène, -SO₃Na ou un groupe alkyle ; et
4) des dérivés de triazine-stilbène de formule (IV) :

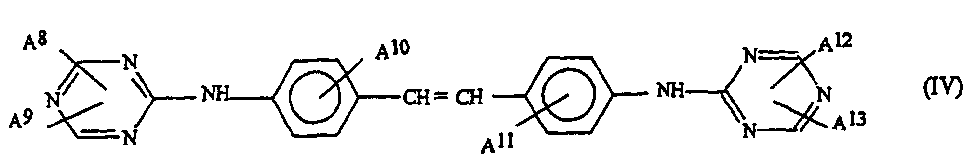

dans laquelle A⁸, A⁹, A¹² et A¹³ représentent indépendamment un atome d'hydrogène, un groupe ―SO₃Na ou un groupe phénylamino, dialkylamino ou morpholino, et A¹⁰ et A¹¹ représentent indépendamment un atome d'hydrogène ou ―SO₃Na.

Dans les formules données ci-dessus, les groupes aromatiques utilisés peuvent être des groupes comportant un ou plusieurs noyaux benzéniques, par exemple des groupes phényle, biphényle, phényle substitués ou des groupes aromatiques formés à partir d'hydrocarbures aromatiques polycycliques, comme les groupes naphtyle, phénanthryle, anthracényle, fluoranthényle, etc.

Les groupes hétérocycliques sont des groupes hydrocarbonés, saturés ou insaturés, comportant un ou plusieurs hétéroatomes tels que O, N et S. A titre d'exemple de groupes hétérocycliques, on peut citer les groupes thiényle, furyle, pyrannyle, isobenzofurannyle, isobenzothiényle, pyrrolyle, pyridyle, pyrazolyle.

Les groupes alcoylène utilisés peuvent être linéaires ou ramifiés et comporter de 3 à 16 atomes de carbone.

Les groupes alkyle susceptibles d'être utilisés sont des groupes linéaires ou ramifiés, ayant de préférence de 1 à 16 atomes de carbone. On utilise en particulier le groupe tert-butyle. Ceci est notamment le cas pour les substituants A⁴ et A⁵ dans la formule (III).

Lorsque dans la formule (IV), A⁸, A⁹, A¹² et/ou A¹³ représentent un groupe dialkylamino, les groupes alkyle ont de préférence de 1 à 12 atomes de carbone. A titre d'exemple, l'azurant optique peut répondre à la formule (III) dans laquelle A⁴ et A⁵ représentent un groupe alkyle, par exemple le groupe tert-butyle et A⁶, A⁷, B¹ et B² représentent un atome d'hydrogène.

On peut aussi utiliser un azurant optique répondant à la formule (I) dans laquelle A¹ représente le groupe : 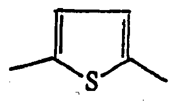
et A² représente le groupe tert-butyle.

On peut encore utiliser un azurant optique répondant à la formule (IV) dans laquelle A¹⁰ et A¹¹ représentent -SO₃Na et A⁸, A⁹, A¹² et A¹³ représentent le groupe phénylamino.

Dans le copolymère de l'invention, les motifs A et B peuvent être issus de divers monomères polymérisables de structures similaires ou différentes.

Ainsi, la succession des motifs A et B peut correspondre au squelette d'un polymère naturel tel que les hydroxyéthers de cellulose, les polysaccharides, les esters de cellulose, des protéines modifiées et des hydrolysats de protéines. De préférence, les motifs A et B sont des motifs de polymères synthétiques, par exemple des motifs issus de monomères acryliques, méthacryliques, vinyliques, oléfiniques et/ou styréniques.

Selon un mode de réalisation particulier, le copolymère comprend des motifs A issus du méthacrylate de méthyle et des motifs B issus du méthacrylate de l'azurant optique.

A titre d'exemple, le copolymère peut répondre à la formule (V) : 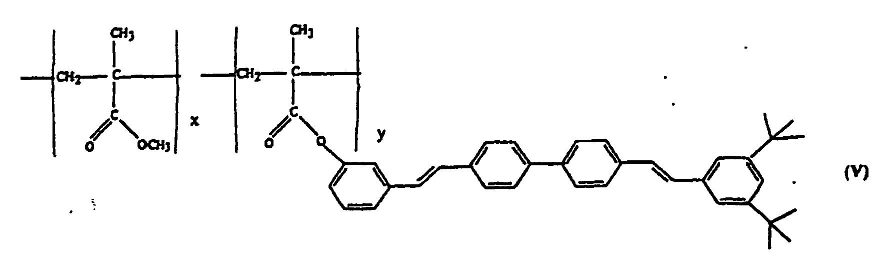
dans laquelle x et y sont tels que la proportion massique de groupes azurants optiques C de formule (VI) : 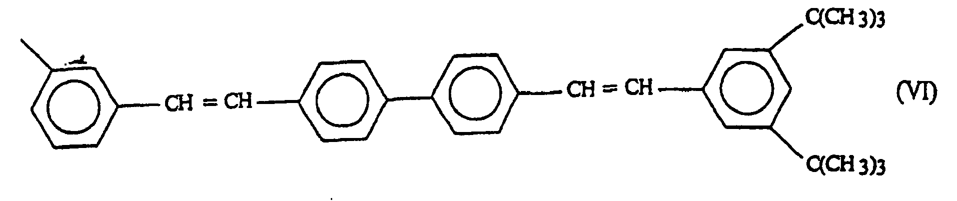
représente de 4 à 30% du poids total du copolymère.

Dans cette formule, les motifs A sont formés du méthacrylate de méthyle et les motifs B du méthacrylate de l'azurant optique. Ils sont répartis de façon aléatoire dans la chaîne du copolymère.

De préférence, la proportion massique de groupes azurants optiques C représente 8 à 10% en poids du copolymère total. Cette limitation de la quantité d'azurant optique permet de diminuer le coût du copolymère utilisé selon l'invention, et par conséquent de la composition elle-même, tout en obtenant l'effet maximal de fluorescence réémise.

Les copolymères de l'invention peuvent être préparés par des procédés classiques, par exemple par copolymérisation radicalaire d'un monomère A' et d'un monomère B' sur lequel est fixé l'azurant optique C par une liaison covalente. Cette copolymérisation radicalaire est généralement effectuée en solution organique en présence d'un amorceur de polymérisation tel que l'azoisobutyronitrile. On peut aussi préparer le copolymère de l'invention par le procédé décrit dans la demande FR 99/10942.

La composition selon l'invention trouve une application dans le soin et le maquillage de la peau, des muqueuses et des phanères.

L'invention concerne ainsi un procédé cosmétique pour augmenter la clarté et/ou l'éclat de la peau, des muqueuses ou des phanères, caractérisé par le fait que l'on applique sur la peau, les muqueuses ou les phanères une composition telle que définie précédemment.

L'invention concerne également un procédé cosmétique pour augmenter la brillance des cheveux, caractérisé par le fait que l'on applique sur les cheveux une composition telle que définie précédemment.

Ces applications seront maintenant détaillées.

L'invention a en particulier pour objet l'utilisation de la composition précitée comme composition cosmétique, ou pour la préparation d'une composition dermatologique, pour application topique sur la peau, en vue de blanchir la peau et/ou conférer au teint une plus grande uniformité, une plus grande transparence et/ou un aspect de porcelaine. Une telle utilisation est particulièrement efficace sur les peaux asiatiques, bien qu'elle convienne également aux peaux caucasiennes.

Lorsque la composition selon l'invention est une composition de soin de la peau, la composition selon l'invention comprend en outre de préférence au moins un agent dépigmentant. Cette association permet de combiner l'effet blanchissant à long terme de l'agent dépigmentant, par inhibition de la synthèse de mélanine, et l'effet blanchissant immédiat de l'azurant optique.

Comme agent dépigmentant, les compositions selon l'invention peuvent par exemple comprendre au moins l'un des composés suivants : l'acide kojique ; l'acide ellagique ; l'arbutine et ses dérivés tels que ceux décrits dans les demandes EP-895 779 et EP-524 109 ; l'hydroquinone ; les dérivés d'aminophénol tels que ceux décrits dans les demandes WO 99/10318 et WO 99/32077, et en particulier le N-cholestéryloxycarbonyl-para-aminophénol et le N-éthyloxycarbonyl-para-aminophénol ; les dérivés d'iminophénol, en particulier ceux décrits dans la demande WO 99/22707 ; l'acide L-2-oxothiazolidine-4-carboxylique ou procystéine, ainsi que ses sels et esters ; l'acide ascorbique et ses dérivés, notamment le glucoside d'ascorbyle ; et les extraits de plantes, en particulier de réglisse, de mûrier et de scutellaire, sans que cette liste soit limitative.

De préférence, une telle composition comprend en outre au moins un agent susceptible de filtrer les UV et/ou au moins un agent desquamant. A cet égard, le copolymère selon l'invention peut lui-même être considéré comme un absorbeur UV, de sorte que son association avec un filtre UV classique permet d'additionner les effets de ces deux composés et éventuellement de réduire la quantité de filtre UV nécessaire à l'obtention d'un Facteur de Protection Solaire donné. Cela présente un avantage, dans la mesure où les filtres UV ont une forte influence sur la rhéologie des compositions les contenant, de sorte qu'ils restreignent la liberté de formulation de ces compositions en terme de texture.

Parmi les agents susceptibles de filtrer les UV, on peut citer les filtres organiques hydrophiles ou lipophiles et les filtres minéraux.

Les filtres hydrophiles peuvent être notamment choisis parmi les dérivés de la benzophénone, les dérivés de l'acide p-aminobenzoïque, les dérivés du camphre ou encore parmi les dérivés de benzimidazole.

Les filtres hydrophiles peuvent être présents dans la composition finale selon l'invention à une teneur pouvant varier de 0,1 à 20 %, de préférence de 0,2 à 10 %, en poids, par rapport au poids total de la composition.

Les filtres lipophiles convenant particulièrement bien à la présente invention peuvent être choisis parmi les dérivés du dibenzoylméthane, les dérivés de benzimidazole, les dérivés cinnamiques, les dérivés salicyliques, les dérivés du camphre, les dérivés de triazine, les dérivés de la benzophénone, les dérivés de β,β-diphénylacrylate, les dérivés de l'acide p-aminobenzoïque, les polymères filtres et les silicones filtres décrits dans les demandes WO-93/04665 et WO-94/06404. D'autres exemples de filtres organiques sont donnés dans la demande de brevet EP-A 0 487 404.

Le ou les filtres lipophiles peuvent être présents dans la composition selon l'invention à une teneur pouvant varier de 0,5 à 30 %, de préférence de 0,5 à 20 %, en poids, par rapport au poids total de la composition.

Les filtres minéraux peuvent être des pigments ou bien encore des nanopigments (taille moyenne des particules primaires : généralement entre 5 nm et 150 nm, de préférence entre 10 et 100 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflection et/ou diffusion) du rayonnement UV. Des agents d'enrobage classiques sont par ailleurs l'alumine et/ou le stéarate d'aluminium, les silicones. De tels nanopigments d'oxydes métalliques, enrobés ou non enrobés, sont en particulier décrits dans les demandes de brevets EP-A- 0 518 772 et EP-A-0 518 773.

Les filtres minéraux peuvent être présents dans la composition finale selon l'invention à une teneur pouvant varier de 0,1 à 20 %, de préférence de 0,2 à 10 % en poids, par rapport au poids total de la composition.

Les agents desquamants susceptibles d'être utilisés dans les compositions selon l'invention comprennent notamment les α-hydroxyacides tels que les acides citrique, lactique, glycolique, mandélique, malique et tartrique ; les β-hydroxyacides et notamment l'acide salicylique et ses dérivés décrits dans les demandes FR-A-2 581 542, EP-875 495, WO 98/35973 et EP-756 866; les α-cétoacides et les β-cétoacides ; les rétinoïdes et en particulier le rétinol et les esters de rétinyle ; les inhibiteurs d'HMG-CoA réductase, comme décrit dans la demande EP-738 510 ; et les dérivés de sucre tels que ceux décrits dans la demande EP-853 472, et en particulier le O-octanoyl-6'-β-D-maltose.

Lorsque la composition selon l'invention est une composition de soin ou de maquillage de la peau, il peut s'agir d'une composition cosmétique de prévention et/ou de traitement des cernes. Les cernes se manifestant par la présence d'une surface plus foncée, voir grise ou noire, autour des yeux et/ou par la présence de poches sous les yeux et/ou par un aspect flétri du contour des yeux peuvent ainsi diminuer, voire disparaître, grâce à l'application sur ceux-ci de la composition selon l'invention.

Une telle composition particulièrement efficace dans la prévention et/ou le traitement des cernes comprend de préférence en outre au moins un agent hydratant.

Parmi les agents hydratants susceptibles d'être utilisés dans ces compositions, on peut citer en particulier l'urée ou ses dérivés, les polyols, comme par exemple la glycérine, le sorbitol, le propylène glycol.

Lorsque la composition selon l'invention est une composition de maquillage de la peau, il peut en variante s'agir d'une composition de fond de teint ou de poudre.

L'invention a également pour objet l'utilisation de la composition précitée comme composition de soin ou de maquillage des phanères ou des muqueuses. En particulier, l'invention a pour objet l'utilisation de la composition précitée comme stick à lèvres (coloré ou non coloré), mascara ou vernis à ongles, pour augmenter la clarté et/ou l'éclat des lèvres, des cils ou des ongles.

L'invention a encore pour objet l'utilisation de la composition précitée comme composition capillaire, destinée en particulier à augmenter la brillance des cheveux et/ou à modifier leurs reflets.

La composition selon l'invention peut également permettre de modifier la couleur des lèvres, des cils et des ongles dans le cas où elle renferme un ou plusieurs pigments colorés.

Le copolymère précédemment décrit est utilisé dans la composition selon la présente invention de préférence en quantité allant de 0,1 à 15% en poids, et encore plus préférentiellement de 0,5 à 10% en poids par rapport au poids total de la composition.

Les compositions selon l'invention peuvent présenter toutes les formes galéniques normalement utilisées pour une application topique, par exemple sous forme de solutions, de gels, de dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel ou stick, ou encore de microémulsions, de microcapsules, de microparticules ou de dispersions vésiculaires de type ionique et/ou non ionique. Ces compositions sont préparées selon les méthodes usuelles.

De façon connue, les compositions, plus particulièrement cosmétiques, de l'invention peuvent contenir des adjuvants habituels dans le domaine cosmétique, tels que les émulsionnants, les gélifiants hydrophiles ou lipophiles, les actifs hydrophiles ou lipophiles, les conservateurs, les antioxydants, les parfums, les charges, les matières colorantes. Les quantités de ces différents adjuvants sont celles classiquement utilisées dans le domaine cosmétique, et par exemple de 0,01 à 20 % du poids total de la composition. Ces adjuvants, selon leur nature, peuvent être introduits dans la phase grasse, dans la phase aqueuse et/ou dans les vésicules lipidiques.

Comme émulsionnants, on peut utiliser des émulsionnants eau-dans-huile (E/H) ou huile-dans-eau (H/E) selon l'émulsion finale souhaitée.

Comme émulsionnants, on peut citer par exemple le mélange de stéarate de glycéryle/stéarate de PEG-100 (Arlacel 165 vendu par la société ICI), le stéarate de PEG-20 (Myrj 49 vendu par la société ICI), le stéarate de PEG-40 (Myrj 52 vendu par la société ICI) et le tristéarate de sorbitan (Span 65 vendu par la société ICI).

Le taux d'émulsionnant peut aller de 0,1 à 15 % en poids, et de préférence de 0,5 à 5 % en poids, par rapport au poids total de la composition.

On peut ajouter à la composition selon l'invention des coémulsionnants, par exemple en une quantité allant de 0,05 % à 10 % en poids par rapport au poids total de la composition.

Dans les dispersions de vésicules lipidiques, l'émulsionnant peut être constitué par les vésicules elles-mêmes, de lipides ioniques et/ou non ioniques.

Comme huiles utilisables dans l'invention, on peut citer les huiles minérales, les huiles végétales (huile de germe de maïs), les huiles de synthèse (isohexadécane), les huiles siliconées (cyclométhicone) et les huiles fluorées. On peut utiliser aussi des alcools gras (alcool stéarylique, alcool cétylique), des acides gras (acide stéarique) et des cires.

Comme actifs hydrophiles, on peut utiliser par exemple les protéines ou les hydrolysats de protéine, les acides aminés, l'urée, l'allantoïne, les sucres et leurs dérivés, l'acide glycyrrhétinique.

Comme actifs lipophiles, on peut utiliser le tocophérol (vitamine E) et ses dérivés, les acides gras essentiels, les céramides, les huiles essentielles.

Les exemples suivants illustrent l'invention. Dans ces exemples, les proportions indiquées sont des pourcentages en poids.

### Exemple 1 : Crème fluide H/E

On prépare de façon classique la composition suivante.
- Palmitate d'éthyl-2 hexyle 8 %
- Huile de vaseline 8 %
- Mono-di-isostéarate de glycéryle 2 %
- Mélange de mono-di-stéarate de glycéryle, acide stéarique, glycérine (40/50/5/5) 2 %
- Copolymère (V) * 1 %
- Triéthanolamine 0,9 %
- Propylène glycol 2 %
- Acide stéarique 2 %
- Stéarate de magnésium 2 %
- Conservateurs qs
- Eau qsp 100 %
* comprenant environ 9% en poids d'azurant optique par rapport au poids total du copolymère (y = 0,02).

Cette crème confère à la peau un aspect de porcelaine.

### Exemple 2 : Anti-cernes

On prépare de façon classique la composition suivante.
- Hyaluronate de sodium 0,1 %
- Copolymère (V) * 5 %
- Acide acrylamidométhyl propane sulfonique neutralisé partiellement à l'ammoniaque réticulé 1,5 %
- Glycérine 5 %
- Alcool éthylique 5 %
- Di-oléate de méthyl glucose oxyéthyléné (120 OE) 0,5 %
- Mono-laurate de sorbitane oxyéthyléné (20 OE) 0,5 %
- Conservateurs qs
- Eau qsp 100 %
* comprenant environ 9% en poids d'azurant optique par rapport au poids total du copolymère (y = 0,02).

Cette composition permet d'estomper l'apparence des cernes.

### Exemple 3 : Rouge à lèvres

On prépare, de façon classique, la composition suivante.
- Polybutène 15 %
- Benzoate d'alkyle en C₁₂-C₁₅ 20 %
- Trimellitate de tridécyle 15 %
- Phényltriméthicone 5 %
- Di-isostéarylmalate 7 %
- Lanoline 13 %
- Cire de polyéthylène 4 %
- Cire de candellila 7 %
- Cire de carnauba 3,9 %
- BHT 0,1 %
- Copolymère (V) 10 %
- FD & C Yellow n° 6 Al Lake 1 %
- TiO₂ 0,5 %
- DC Red n° 7 Calcium Lake 0,7 %
* comprenant environ 9% en poids d'azurant optique par rapport au poids total du copolymère (y = 0,02).

Ce stick apporte de la brillance aux lèvres et des reflets changeants sous l'effet des UV.

### Exemple 4 : Après-shampooing

On prépare de façon classique la composition suivante.
- Mélange d'alcool cétéarylique et d'alcool cétéarylique oxyéthyléné (30 OE) 5,0 %
- Chlorure de béhényl triméthyl ammonium 2,0 %
- Amodiméthicone 1,2%
- Copolymère (V)* 0,8 %
- Carbomer neutralisé 0,5 %
- Eau qsp 100 %
* comprenant environ 9% en poids d'azurant optique par rapport au poids total du copolymère (y = 0,02).

Cet après-shampooing apporte de la brillance aux cheveux.

## Revendications

1. Composition pour application topique comprenant, dans un milieu physiologiquement acceptable, au moins un copolymère comportant au moins un azurant optique fixé sur la chaîne du copolymère par une liaison covalente.

2. Composition selon la revendication 1, **caractérisée en ce que** ledit copolymère est formé de motifs A et de motifs B comportant un azurant optique C lié de façon covalente aux motifs B, et **en ce qu'**il comprend 4 à 30% en poids d'azurant optique C par rapport au poids total du copolymère.

3. Composition selon la revendication 2, **caractérisée en ce que** ledit copolymère comprend de 8 à 10% en poids d'azurant optique C par rapport au poids total du copolymère.

4. Composition selon l'une quelconque des revendications 1 à 3, **caractérisée en ce que** ledit azurant optique est choisi dans le groupe constitué :
1) des bis(benzoxazol-2-yl) de formule (I) : 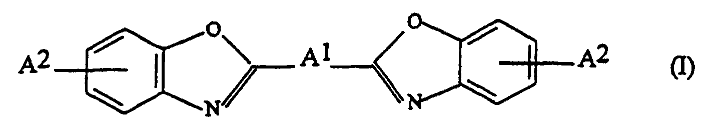
dans laquelle A¹ est un groupe aromatique, hétérocyclique ou alcoylène, et A² est un atome d'hydrogène ou un groupe alkyle ;
2) des coumarines de formule (II) : 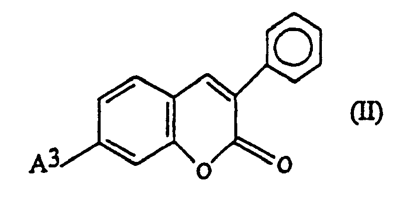
dans laquelle A³ est est un groupe hétérocyclique ;
3) des bis(styryl)biphényle de formule (III) : 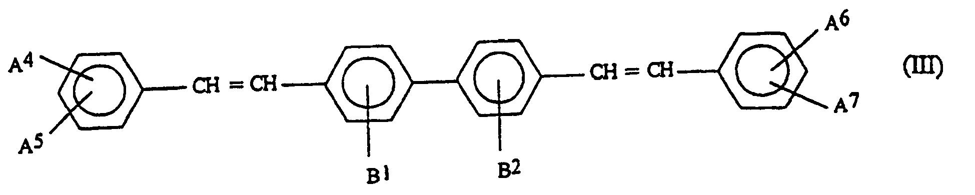
dans laquelle A⁴, A⁵, A⁶, A⁷, B¹ et B², qui peuvent être identiques ou différents, représentent indépendamment un atome d'hydrogène, -SO₃Na ou un groupe alkyle ; et
4) des dérivés de triazine-stilbène de formule (IV) : 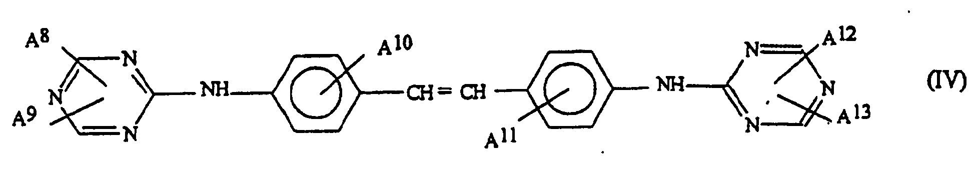
dans laquelle A⁸, A⁹, A¹² et A¹³ représentent indépendamment un atome d'hydrogène, un groupe -SO₃Na ou un groupe phénylamino, dialkylamino ou morpholino, et A¹⁰ et A¹¹ représentent indépendamment un atome d'hydrogène ou -SO₃Na.

5. Composition selon la revendication 4, **caractérisée en ce que** l'azurant optique répond à la formule (III) dans laquelle A⁴ et A⁵ représentent un groupe alkyle et A⁶, A⁷, B¹ et B² représentent un atome d'hydrogène.

6. Composition selon la revendication 5, **caractérisée en ce que** A⁴ et A⁵ représentent le groupe tert-butyle.

7. Composition selon la revendication 4, **caractérisée en ce que** ledit azurant optique répond à la formule (I) dans laquelle A¹ représente le groupe : 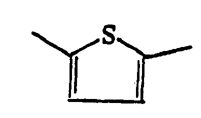 et A² représente le groupe tert-butyle.

8. Composition selon la revendication 4, **caractérisée en ce que** ledit azurant optique répond à la formule (IV) dans laquelle A¹⁰ et A¹¹représentent -SO₃Na et A⁸, A⁹, A¹² et A¹³ représentent le groupe phénylamino.

9. Composition selon l'une quelconque des revendications 2 à 8, **caractérisée en ce que** le motif A est issu du méthacrylate de méthyle et le motif B est issu du méthacrylate de l'azurant optique.

10. Composition selon l'une quelconque des revendications 1 à 6, **caractérisée en ce que** ledit copolymère répond à la formule (V) : 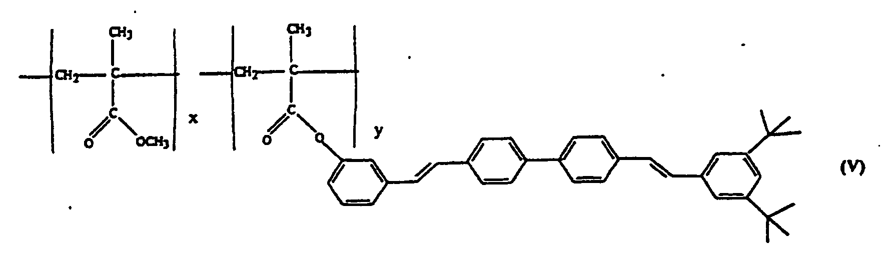
dans laquelle x et y sont tels que la proportion massique de groupes azurants optiques C de formule (VI) : 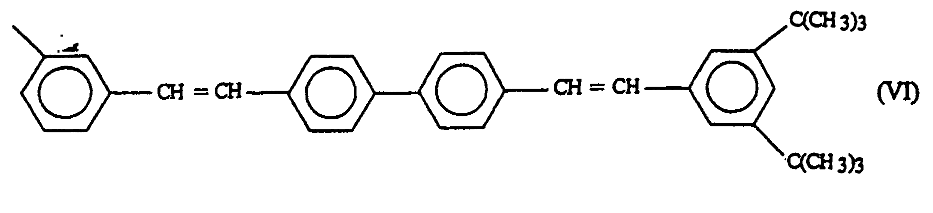
représente de 4 à 30% du poids total du copolymère.

11. Composition selon l'une quelconque des revendications 1 à 10, **caractérisée en ce qu'**elle comprend en outre au moins un agent dépigmentant.

12. Composition selon l'une quelconque des revendications 1 à 11, **caractérisée en ce qu'**elle comprend en outre au moins un agent susceptible de filtrer les UV et/ou au moins un agent desquamant.

13. Composition selon l'une quelconque des revendications 1 à 12, **caractérisée en ce que** ledit copolymère est présent en quantité allant de 0,1 à 15% en poids, et encore plus préférentiellement de 0,5 à 10% en poids, par rapport au poids total de la composition.

14. Utilisation de la composition selon l'une quelconque des revendications 1 à 13 comme composition cosmétique, ou pour la préparation d'une composition dermatologique, pour application topique sur la peau, en vue de blanchir la peau et/ou conférer au teint une plus grande uniformité, une plus grande transparence et/ou un aspect de porcelaine.

15. Utilisation de la composition selon l'une quelconque des revendications 1 à 13 comme composition cosmétique de prévention et/ou de traitement des cernes.

16. Utilisation selon la revendication 15, **caractérisée en ce que** ladite composition comprend en outre au moins un agent hydratant.

17. Utilisation de la composition selon l'une quelconque des revendications 1 à 13 comme composition de fond de teint ou de poudre.

18. Utilisation de la composition selon l'une quelconque des revendications 1 à 13 comme composition de soin ou de maquillage des phanères ou des muqueuses.

19. Utilisation de la composition selon l'une quelconque des revendications 1 à 13 comme stick à lèvres, mascara ou vernis à ongles, pour augmenter la clarté et/ou l'éclat des lèvres, des cils ou des ongles.

20. Utilisation de la composition selon l'une quelconque des revendications 1 à 13 comme composition capillaire destinée à augmenter la brillance des cheveux et/ou à modifier leurs reflets.

21. Procédé cosmétique pour augmenter la clarté et/ou l'éclat de la peau, des muqueuses ou des phanères, **caractérisé par le fait que** l'on applique sur la peau, les muqueuses ou les phanères une composition selon l'une quelconque des revendications 1 à 13.

22. Procédé cosmétique pour augmenter la brillance et/ou modifier les reflets des cheveux, **caractérisé par le fait que** l'on applique sur les cheveux une composition selon l'une quelconque des revendications 1 à 13.

## Patentansprüche

1. Zusammensetzung zur topischen Anwendung, die in einem physiologisch akzeptablen Medium mindestens ein Copolymer enthält, das mindestens einen optischen Aufheller umfasst, der über eine kovalente Bindung an die Kette des Copolymers gebunden ist.

2. Zusammensetzung nach Anspruch 1, **dadurch gekennzeichnet, dass** das Copolymer aus Einheiten A und aus Einheiten B gebildet wird, welche einen optischen Aufheller C enthalten, der kovalent an die Einheiten B gebunden ist, und **dadurch**, dass es 4 bis 30 Gew.-% optischen Aufheller C, bezogen auf das Gesamtgewicht des Copolymers, enthält.

3. Zusammensetzung nach Anspruch 2, **dadurch gekennzeichnet, dass** das Copolymer 8 bis 10 Gew.-% optischen Aufheller C, bezogen auf das Gesamtgewicht des Copolymers, enthält.

4. Zusammensetzung nach einem der Ansprüche 1 bis 3, **dadurch gekennzeichnet, dass** der optische Aufheller unter den folgenden Verbindungen ausgewählt ist:
1) Bis(benzoxazol-2-yl)-Verbindungen der Formel (I): 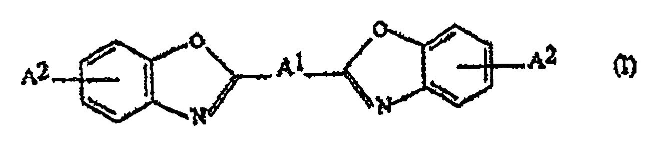
worin A¹ eine aromatische Gruppe, heterocyclische Gruppe oder Alkylengruppe ist, und A² ein Wasserstoffatom oder eine Alkylgruppe bedeutet;
2) Cumarinen der Formel (II); 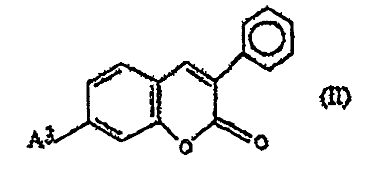
worin A3 eine heterocyclische Gruppe ist.
3) Bis(styryl)biphenylenen der Formel (III): 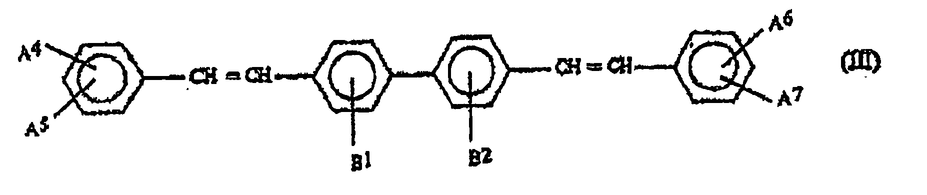
worin A⁴, A⁵, A⁶, A⁷, B¹ und B², die gleich oder verschieden sein können, unabhängig voneinander ein Wasserstoffatom, -SO₃Na oder eine Alkylgruppe bedeuten; und
4) Triazinstilbenderivaten der Formel (IV): 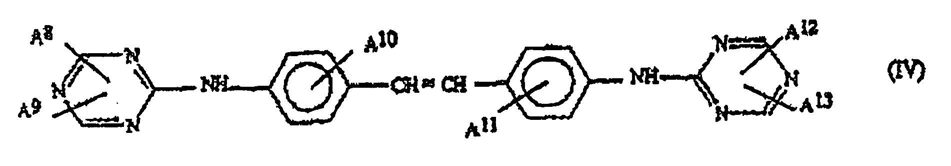
worin A⁸, A⁹, A¹² und A¹³ unabhängig voneinander ein Wasserstoffatom, eine Gruppe -SO₃Na oder eine Gruppe Phenylamino, Dialkylamino oder Morpholino bedeuten, und die Gruppen A¹⁰ und A¹¹ unabhängig voneinander ein Wasserstoffatom oder -SO₃Na bedeuten.

5. Zusammensetzung nach Anspruch 4, **dadurch gekennzeichnet, dass** der optische Aufheller der Formel (III) entspricht, worin A⁴ und A⁵ eine Alkylgruppe bedeuten und A⁶, A⁷, B¹ und B² ein Wasserstoffatom bedeuten.

6. Zusammensetzung nach Anspruch 5, **dadurch gekennzeichnet, dass** A⁴ und A⁵ *t*-Butyl bedeuten.

7. Zusammensetzung nach Anspruch 4, **dadurch gekennzeichnet, dass** der optische Aufheller der Formel (I) entspricht, worin A¹ die folgende Gruppe ist: 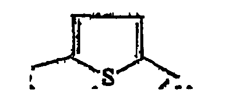
und A² die *t*-Butylgruppe bedeutet.

8. Zusammensetzung nach Anspruch 4, **dadurch gekennzeichnet, dass** der optische Aufheller der Formel (IV) entspricht, worin A¹⁰ und A¹¹ -SO₃Na bedeuten und A⁸, A⁹, A¹² und A¹³ die Phenylaminogruppen bedeuten.

9. Zusammensetzung nach einem der Ansprüche 2 bis 8, **dadurch gekennzeichnet, dass** die Einheit A von Methylmethacrylat abgeleitet ist und die Einheit B von dem Methacrylat des optischen Aufhellers abgeleitet ist.

10. Zusammensetzung nach einem der Ansprüche 1 bis 6, **dadurch gekennzeichnet, dass** das Copolymer der folgenden Formel (V) entspricht: 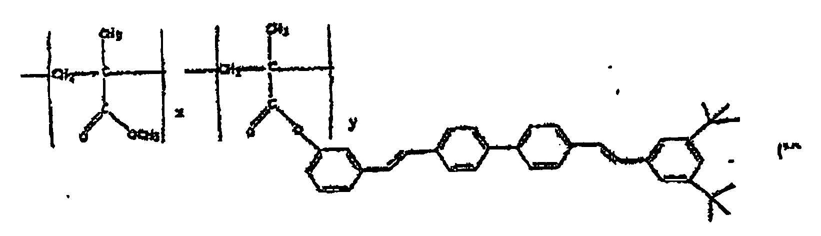
worin x und y so gewählt sind, dass der Masseanteil der optischen Aufhellergruppen C der Formel (VI): 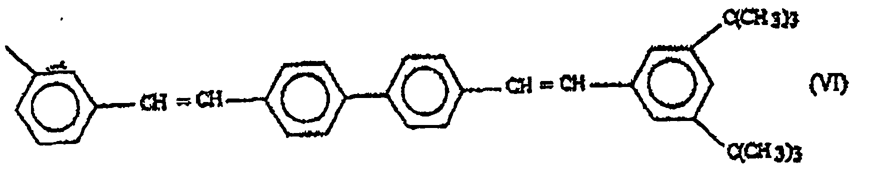
4 bis 30 % des Gesamtgewichts des Copolymers ausmachen.

11. Zusammensetzung nach einem der Ansprüche 1 bis 10, **dadurch gekennzeichnet, dass** sie ferner mindestens ein Depigmentierungsmittel enthält.

12. Zusammensetzung nach einem der Ansprüche 1 bis 11, **dadurch gekennzeichnet, dass** sie ferner mindestens einen Stoff enthält, der die UV-Strahlung filtern kann und/oder mindestens einen abschuppenden Wirkstoff.

13. Zusammensetzung nach einem der Ansprüche 1 bis 12, **dadurch gekennzeichnet, dass** das Copolymer in einer Menge von 0,1 bis 15 Gew.-% und noch bevorzugter 0,5 bis 10 Gew.-%, bezogen auf das Gesamtgewicht der Zusammensetzung, enthalten ist.

14. Verwendung der Zusammensetzung nach einem der Ansprüche 1 bis 13 als kosmetische Zusammensetzung oder für die Herstellung einer dermatologischen Zusammensetzung für die topische Anwendung auf die Haut, um die Haut zu bleichen und/oder dem Teint eine größere Gleichförmigkeit, eine größere Transparenz und/oder ein porzellanartiges Aussehen zu geben.

15. Verwendung der Zusammensetzung nach einem der Ansprüche 1 bis 13 als kosmetische Zusammensetzung zur Vorbeugung und/oder Behandlung von Augenringen.

16. Verwendung nach Anspruch 15, **dadurch gekennzeichnet, dass** die Zusammensetzung ferner mindestens ein Hydratisierungsmittel enthält.

17. Verwendung der Zusammensetzung nach einem der Ansprüche 1 bis 13 als Make-up oder Puder.

18. Verwendung der Zusammensetzung nach einem der Ansprüche 1 bis 13 als Zusammensetzung für die Pflege oder zum Schminken der Hautanhangsgebilde oder Schleimhäute.

19. Verwendung der Zusammensetzung nach einem der Ansprüche 1 bis 13 als Lippenstift, Mascara oder Nagellack, um die Helligkeit und/oder das strahlende Aussehen der Lippen, Wimpern oder Nägel zu verbessern.

20. Verwendung der Zusammensetzung nach einem der Ansprüche 1 bis 13 als Zusammensetzung für die Haarbehandlung, die dazu vorgesehen ist, den Glanz der Haare zu erhöhen und/oder ihre Reflexe zu modifizieren.

21. Kosmetisches Verfahren, um die Helligkeit und/oder das strahlende Aussehen der Haut, der Schleimhäute oder der Hautanhangsgebilde zu erhöhen, **dadurch gekennzeichnet, dass** es darin besteht, auf die Haut, die Schleimhäute oder die Hautanhangsgebilde eine Zusammensetzung nach einem der Ansprüche 1 bis 13 aufzutragen.

22. Kosmetisches Verfahren zur Verbesserung des Glanzes und/ oder zur Modifizierung der Reflexe der Haare, **dadurch gekennzeichnet, dass** auf die Haare eine Zusammensetzung nach einem der Ansprüche 1 bis 13 aufgetragen wird.

## Claims

1. Composition for topical application comprising, in a physiologically acceptable medium, at least one copolymer comprising at least one optical brightener attached to the copolymer chain via a covalent bond.

2. Composition according to Claim 1, **characterized in that** the said copolymer is formed from units A and from units B comprising an optical brightener C bonded covalently to the units B, and **in that** it comprises 4% to 30% by weight of optical brightener C relative to the total weight of the copolymer.

3. Composition according to Claim 2, **characterized in that** the said copolymer comprises from 8% to 10% by weight of optical brightener C relative to the total weight of the copolymer.

4. Composition according to any one of Claims 1 to 3, **characterized in that** the said optical brightener is chosen from the group consisting of:
1) bis(benzoxazol-2-yl) compounds of formula (I): 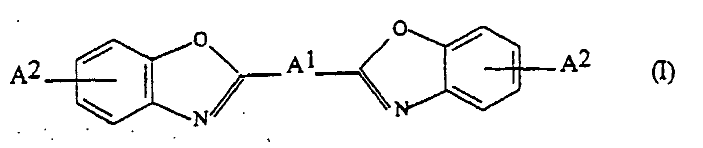
in which A¹ is an aromatic, heterocyclic or alkylene group and A² is a hydrogen atom or an alkyl group;
2) coumarins of formula (II): 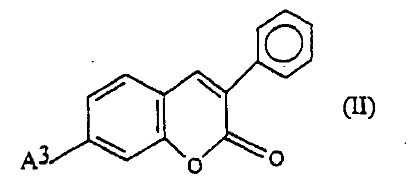
in which A³ is a heterocyclic group;
3) bis(styryl)biphenyl compounds of formula (III): 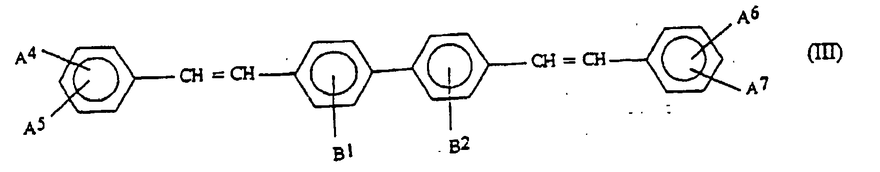
in which A⁴, A⁵, A⁶, A⁷, B¹ and B², which may be identical or different, independently represent a hydrogen atom, -SO₃Na or an alkyl group; and
4) triazine-stilbene derivatives of formula (IV): 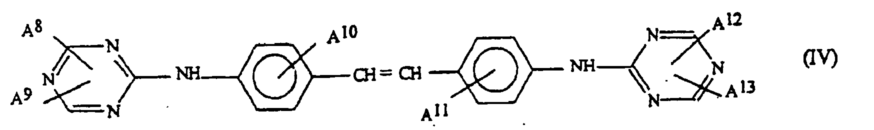
in which A⁸, A⁹, A¹² and A¹³ independently represent a hydrogen atom, an -SO₃Na group or a phenylamino, dialkylamino or morpholino group and A¹⁰ and A¹¹ independently represent a hydrogen atom or -SO₃Na.

5. Composition according to Claim 4, **characterized in that** the optical brightener corresponds to formula (III) in which A⁴ and A⁵ represent an alkyl group and A⁶, A⁷, B¹ and B² represent a hydrogen atom.

6. Composition according to Claim 5, **characterized in that** A⁴ and A⁵ represent a tert-butyl group.

7. Composition according to Claim 4, **characterized in that** the said optical brightener corresponds to formula (I) in which A¹ represents a group: 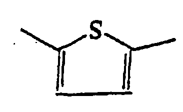 and A² represents a tert-butyl group.

8. Composition according to Claim 4, **characterized in that** the said optical brightener corresponds to formula (IV) in which A¹⁰ and A¹¹ represent ―SO₃Na and A⁸, A⁹, A¹² and A¹³ represent a phenylamino group.

9. Composition according to any one of Claims 2 to 8, **characterized in that** the unit A is derived from methyl methacrylate and the unit B is derived from the methacrylate of the optical brightener.

10. Composition according to any one of Claims 1 to 6, **characterized in that** the said copolymer corresponds to formula (V): 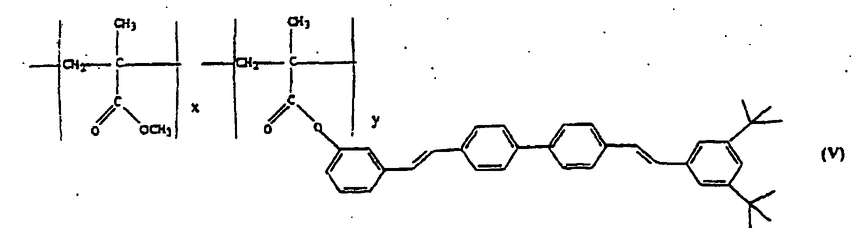
in which x and y are such that the proportion by mass of optical brightener groups C of formula (VI): 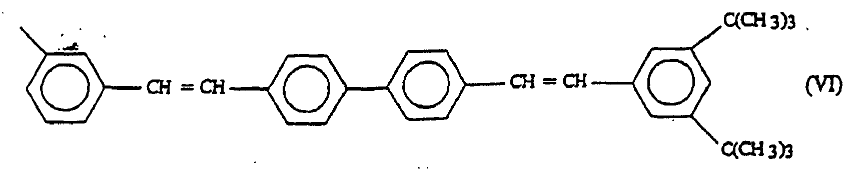
represents from 4% to 30% relative to the total weight of the copolymer.

11. Composition according to any one of Claims 1 to 10, **characterized in that** it also comprises at least one depigmenting agent.

12. Composition according to any one of Claims 1 to 11, **characterized in that** it also comprises at least one agent capable of screening out UV and/or at least one desquamating agent.

13. Composition according to any one of Claims 1 to 12, **characterized in that** the said copolymer is present in an amount ranging from 0.1% to 15% by weight, and even more preferably from 0.5% to 10% by weight, relative to the total weight of the composition.

14. Use of the composition according to any one of Claims 1 to 13 as a cosmetic composition or for the preparation of a dermatological composition, for topical application to the skin, in order to bleach the skin and/or to give the complexion greater uniformity, greater transparency and/or the appearance of porcelain.

15. Use of the composition according to any one of Claims 1 to 13 as a cosmetic composition for preventing and/or treating dark rings under the eyes.

16. Use according to Claim 15, **characterized in that** the said composition also comprises at least one moisturizer.

17. Use of the composition according to any one of Claims 1 to 13 as a foundation or powder composition.

18. Use of the composition according to any one of Claims 1 to 13 as a care or make-up composition for superficial body growths or mucous membranes.

19. Use of the composition according to any one of Claims 1 to 13 as a lipstick, mascara or nail varnish, to increase the brightness and/or radiance of the lips, the eyelashes or the nails.

20. Use of the composition according to any one of Claims 1 to 13, as a haircare composition intended to increase the sheen of the hair and/or to modify its glints.

21. Cosmetic process for increasing the brightness and/or radiance of the skin, mucous membranes or superficial body growths, **characterized in that** a composition according to any one of Claims 1 to 13 is applied to the skin, mucous membranes or superficial body growths.

22. Cosmetic process for increasing the sheen and/or for modifying the glints of the hair, **characterized in that** a composition according to any one of Claims 1 to 13 is applied to the hair.
